# EUROPÄISCHE PATENTANMELDUNG

(11) **EP 2 378 449 A1**
(43) Veröffentlichungstag der Anmeldung: **19.10.2011**
(21) Anmeldenummer: 10003940.3
(22) Anmeldetag: 14.04.2010
(51) Int. Cl.: G06F 19/00, H04N 9/804

(54) **Bildgebendes Diagnosegerät**

(71) Anmelder: Linked IP GmbH, 5081 Anif (AT)
(72) Erfinder: Dr. Golser Rudolf, 5400 Hallein (AT)
(74) Vertreter: von Puttkamer · Berngruber

(57) **Zusammenfassung**

Bei einem bildgebenden Diagnosegerät (1) mit einem PC (2) mit einer Grafikkarte (3), einem Schacht (9) zur Aufnahme eines optischen Laufwerks, einer Schnittstelle (6) zum Anschluss einer Untersuchungsvorrichtung (7) und einem Bildschirm (4) zur Darstellung der mit der Untersuchungsvorrichtung (7) erhaltenen Signale als bewegte Bilder ist in dem Schacht (9) des PC (2) zur Aufnahme eines optischen Laufwerks ein Modul (10) angeordnet, das einen Splitter (12), einen digitalen Signalprozessor (14) zur Datenreduktion und wenigstens einen Speicher (16, 17, 18) aufweist, wobei die Grafikkarte (3) mit dem Splitter (12) verbunden ist und die von der Grafikkarte (13) des PC (2) an den Splitter (12) übertragenen Daten von dem Splitter (12) einerseits dem Bildschirm (4) und andererseits dem digitalen Signalprozessor (14) zugeführt werden, von dem der reduzierte Datenstrom an den Speicher (16, 17, 18) zur Aufzeichnung der bewegten Bilder übertragen wird.

## Beschreibung

Die Erfindung bezieht sich auf ein bildgebendes Diagnosegerät nach dem Oberbegriff des Patentanspruchs 1.

So wird z.B. die Sonographie als bildgebendes Verfahren in der Medizin zur Untersuchung, insbesondere zur Pränataldiagnostik angewendet. Des weiteren werden auch andere bildgebende Verfahren wie die Endoskopie oder die Radiologie mit einem derartigen Diagnosegerät ausgeführt.

Die bekannten bildgebenden Diagnosegeräte verfügen typischerweise über eine PC - basierende Infrastruktur, an welche ein Bedienpanel mit integrierter Tastatur und ein Bildschirm zur Darstellung der gewonnenen Bilddaten ausgeschlossen sind und die eine Schnittstelle zum Anschluss einer Untersuchungsvorrichtung, also beispielsweise einer Ultraschallsonde oder eines Sensors bei radiologischen Verfahren aufweist.

Die von der Untersuchungsvorrichtung empfangenen Signale werden von dem Diagnosegerät mittels aufwendiger Verfahren in sich bewegende 2D bzw. 3D-Modelle umgewandelt, welche schließlich auf dem Bildschirm als bewegte Bilder dargestellt werden. Da die bewegten Bilder mit dem optischen Laufwerk des PCs nicht direkt aufgezeichnet werden können, weil kein unmittelbarer Zugriff auf die eigentlichen Bilddaten besteht, wird bei den bekannten Diagnosegeräten zur Aufnahme und Wiedergabe des Videos ein digitaler Videorecorder, welcher meist als externes Zusatzgerät ausgeführt ist, eingesetzt. Dieser wird dabei über einen zusätzlich notwendigen Ausgang des PC basierenden Systems mit den Bilddaten versorgt, welche typischerweise nur als analoges Videosignal in niedriger Auflösung (z.B. S-Video,...) vorliegen.

Der in den Geräten installierte DVD - Brenner wird zum Abspeichern von Daten verwendet, wobei auch dieser Vorgang aus Performancegründen bzgl. der eingesetzten PC - basierenden Infrastruktur nicht während der Untersuchung durchgeführt werden kann.

Durch das so notwendige, externe Zusatzgerät entstehen höhere Kosten und ein zusätzlicher Platzbedarf. Zudem führen die externen Kabelverbindungen zwischen dem PC und dem digitalen Videorecorder zu den üblichen Problemen externer Kabel.

Aufgabe der Erfindung ist es daher, ein einfach aufgebautes Diagnosegerät bereitzustellen, mit dem die mit der Untersuchungsvorrichtung erhaltenen bewegten Bilder basierend auf dem hochqualitativen Bildsignal des Systemmonitors in nativer Auflösung und ohne Beeinträchtigung der Rechenleistung des PC basierenden Systems im bildgebenden Diagnosegerät aufgenommen und wiedergegeben werden können.

Dies wird erfindungsgemäß mit dem im Anspruch 1 gekennzeichneten Diagnosegerät erreicht. In den Unteransprüchen sind vorteilhafte Ausgestaltungen der Erfindung wiedergegeben.

Nach der Erfindung wird das bestehende optische Laufwerk samt Gehäuse in dem dafür vorgesehenen 5.25" Schacht des PC - basierenden Systems durch ein Modul ersetzt, das in diesen Schacht eingebaut wird und dazu vorzugsweise ein den ursprünglichen Maßen des optischen Laufwerks des Systems entsprechendes Gehäuse aufweist. Statt einem Standard 5,25" Laufwerk wird ein Slim - Line Laufwerk verwendet, der so zusätzlich gewonnene Platz enthält die Elektronik und die für die Modulausführung notwendigen Anschlüsse.

Das Modul weist einen Splitter, einen digitalen Signal Prozessor zur Video Enkodierung (Datenreduktion) und wenigstens einen Speicher zur Aufzeichnung der mit der Untersuchungsvorrichtung generierten, bewegten Bilder auf. Dabei ist die Grafikkarte mit dem Splitter verbunden. Die von der Grafikkarte des PC - basierenden Systems an den Splitter übertragenen Daten werden von dem Splitter einerseits weiter an den Bildschirm des PC - basierenden Systems und andererseits an den digitalen Signalprozessor zur Weiterverarbeitung übertragen. Die Weiterleitung des Bildsignals zum Monitor des PC - basierenden Systems durch den Splitter entfällt typischerweise dann, wenn für die Aufnahme der Videodaten ein zusätzlicher Videoausgang im Gerät bereit gestellt wird und der Monitor direkt mit der Grafikkarte verbunden bleibt. Dies ist z.B. bei tragbaren und Notebook -ähnlichen Geräten dann der Fall, wenn diese über einen zusätzlichen Ausgang für den Anschluss eines externen Monitors aufweisen. Die von dem digitalen Signalprozessor reduzierte Datenmenge wird über die im Modul befindliche zusätzliche CPU dem Speicher zur Aufzeichnung der bewegten Bilder zugeführt und/oder über eine Netzwerkverbindung direkt als Streaming-Video zur Verfügung gestellt.

Zum Anschluss der Grafikkarte an den Splitter des Moduls ist vorzugsweise eine interne Kabelverbindung in dem PC - basierenden System vorgesehen. Dabei kann ein analoges oder digitales Standard-Kabel verwendet werden, beispielsweise ein DVI-, VGA-Kabel oder S-Video Kabel.

Desgleichen ist der Bildschirm des PC mit dem Splitter vorzugsweise durch eine interne Kabelverbindung zwischen Modul und Bildschirm verbunden, wobei auch diese Kabelverbindung vorzugsweise ein analoges oder digitales Standard-Kabel, also beispielsweise ein DVI- oder VGA-Kabel ist. Erfindungsgemäß ist also die Grafikkarte nicht direkt sondern über den Splitter mit dem Bildschirm unter Verwendung von zwei Kabeln verbunden.

Vorzugsweise ist an den Splitter im Modul ein Down-Scaler angeschlossen, um die Eingangsbildgröße a die Anforderungen des jeweiligen Videoaufzeichnungsverfahrens und der Parametrierung in Bezug auf die Bildauflösung anzupassen und gegebenenfalls so auch schon die an den digitalen Signalprozessor übertragene Datenmenge herabzusetzen. Hierbei ist es insbesondere auch möglich, relevante Bildbereiche für die Weiterverarbeitung auszuschneiden. D.h. der Down-Scaler wandelt die hohe Auflösung der Bildsignale des Splitters typischerweise in eine niedrigere Auflösung um, also beispielsweise von SXGA (1280x1024) auf das D1-Format (PAL 720 x 576 bzw. NTSC 720 x 480).

Gleichzeitig ist es mit dem erfindungsgemäß Modul möglich, die Bilddaten in hochauflösender Originalauflösung abzuspeichern und zur Verfügung zu stellen. Dies ist insbesondere auch für die Verwendung neuartiger Datenträgermedien wie Blue-Ray oder HD-DVD von Bedeutung. Auch kann erfindungsgemäß ein an ein Modul angesteckter Wechseldatenträger vorgesehen sein.

Mit dem digitalen Signal Prozessor des Moduls wird der Videodatenstrom durch entsprechende Verfahren wie MPEG-2, MPEG-4 oder H.264 enkodiert, d.h. in einen komprimierten Videodatenstrom umgewandelt, wobei sich die Datenmenge des Videostroms auf z.B. 4 oder 8 MBit / s reduziert.

Die von dem digitalen Signalprozessor komprimierten Videosignale werden vorzugsweise einer CPU, also einem weiteren Prozessor zugeführt, welche zugleich die zentrale Verarbeitungseinheit des Moduls darstellt, also insbesondere die komprimierten Videosignale zur Aufzeichnung auf den verschiedenen Speichermedien weiter verarbeitet.

Diese CPU kann dabei einen Teil des digitalen Signalprozessors bilden oder eine von dem digitalen Signalprozessor räumlich getrennte Komponente in dem Modul sein.

Der Speicher zur Aufzeichnung der von der CPU weiter verarbeiteten Videosignale kann ein flüchtiger und/oder nicht flüchtiger Speicher, also beispielsweise ein SDRAM oder Flash Disk- Speicher sein. Dieser Speicher kann fix im Modul verbaut oder aber auch extern an das Modul als Wechselmedium angesteckt werden (Bsp. USB - Mass Storage Device). Zusätzlich zu diesen Speichervarianten verfügt das Modul vorzugsweise über ein optisches Datenspeichersystem z.B. in Form eines DVD oder Blue Ray Brenners, welches vorzugsweise als Slim-Line Laufwerk ausgebildet wird.

Ferner ist das Modul vorzugsweise mit einer Standard - Schnittstelle wie z. B. USB versehen, damit es mit dem PC basierenden System verbunden werden kann.

Mit dem USB-Datenkabel können damit Befehle vom PC - basierenden System, welche beispielsweise von der Bedieneinheit / Tastatur oder von der auf dem PC -basierenden System laufenden Applikationssoftware kommen, an das Modul übertragen werden (Record, Stop, Pause, ...). Zudem kann der PC über das USB-Kabel auf die in den Datenspeichern des Moduls abgelegten Daten zugreifen, wobei so das übliche optische Laufwerk des PC - basierenden Systems durch das Modul ersetzt wird. Im Gegensatz zu den üblichen optischen Laufwerken im PC basierenden System wird das optische Laufwerk im Modul jedoch völlig autonom durch die lokal verfügbare Recheneinheit (CPU) des Moduls gesteuert.

Nach der Erfindung wird damit ein bildgebendes Diagnosegerät bereit gestellt, bei dem durch das Modul in dem für das optische Laufwerk des PC vorgesehenen Schacht zusätzliche externe Kabelverbindungen entfallen.

Dabei können erfindungsgemäß die üblichen Untersuchungsvorrichtungen an die dafür vorgesehene Schnittstelle des Diagnosegeräts angeschlossen werden, also beispielsweise zur Sonographie eine Ultraschallsonde oder z.B. bei radiographischen Verfahren, wie beim Röntgen entsprechende Sensoren, z.B. mit CCDs.

Die Datenverbindung von dem Modul nach außen kann auch drahtlos sein. Optional kann das Modul auch als externes Gerät verwendet werden. Nachstehend ist die Erfindung anhand der beigefügten Zeichnung beispielhaft näher erläutert.

Darin zeigen:
Figur 1 schematisch ein Diagnosegerät und das Modul, das in dem Schacht angeordnet ist, der an sich zur Aufnahme des optischen Laufwerks des PC dient;
Figur 2 eine perspektivische Ansicht einer bestückten Leiterplatte des Moduls; und
Figur 3 eine perspektivische Ansicht eines Moduls.

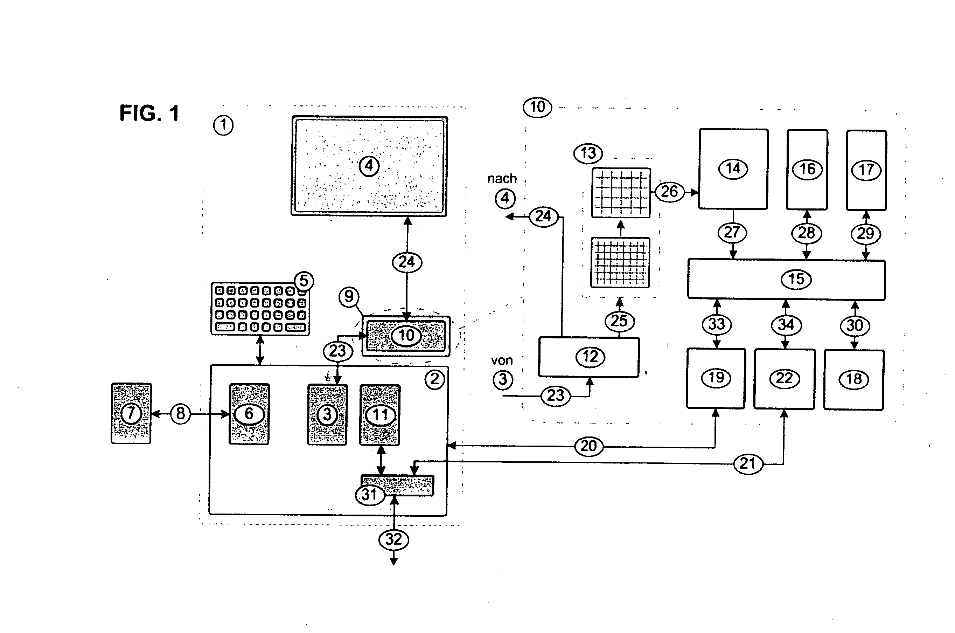

Wie insbesondere aus Figur 1 ersichtlich, weist das Diagnosegerät 1 eine PC basierende Infrastruktur 2 mit einer darin angeordneten Grafikkarte 3, einen Bildschirm 4 und einer Tastatur 5 auf. Der PC 2 ist mit einer Schnittstelle 6 versehen, um eine Untersuchungsvorrichtung 7, z.B. eine Ultraschallsonde, mit einem Kabel 8 anzuschließen.

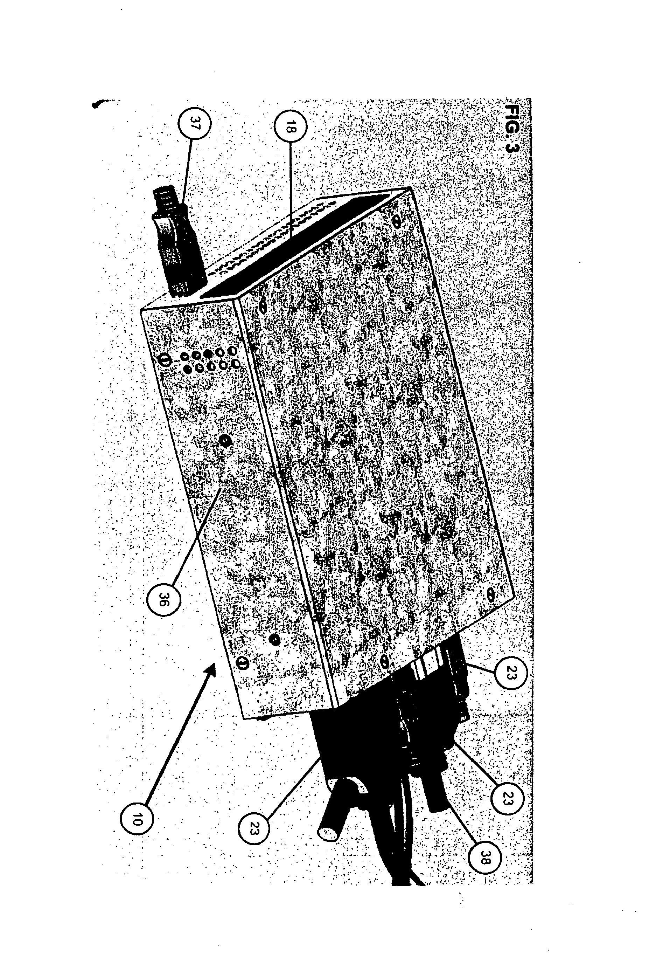

In dem Schacht 9, der in dem Diagnosegerät 1 zur Aufnahme eines optischen Laufwerks vorgesehen ist, ist erfindungsgemäß das Modul 10 angeordnet. Das Modul 10 weist dazu, wie aus Figur 3 ersichtlich, ein Gehäuse 36 auf, das den Abmessungen des optischen Laufwerks des Diagnosegeräts 1 zumindest insoweit entspricht, dass es von dem Schacht 9 aufgenommen werden kann.

Das Modul 10 weist einen Splitter 12, einen Down Sealer 13, einen digitalen Signalprozessor 14, eine CPU 15, einen flüchtigen Speicher 16, einen nicht flüchtigen Speicher 17, ein optisches Laufwerk 18 und eine Schnittstelle 19 im Anschluss eines durch den Pfeil 20 dargestellten USB-Kabels und/oder für eine durch die Schnittstelle 22 und den Pfeil 21 dargestellte Ethernet-Verbindung (LAN) auf. Die Netzwerkkarte des Diagnosegeräts 11 und die Ethernet-Verbindung des Moduls 21 ist dabei mit einem HUB oder Switch 31 verbunden, welcher durch die Kabel- oder Funkverbindung 32 direkt mit dem übergeordneten Netzwerkinfrastruktur / WAN oder Internet verbunden ist.

Der Splitter 12 des Moduls 10 ist mit der Grafikkarte 3 des PC - basierenden Systems 2 durch ein durch den Pfeil 23 dargestelltes analoges oder digitales Standard-Kabel verbunden. Die von der Grafikkarte 3 des PC - basierenden Systems 2 empfangenen Daten werden von dem Splitter 12 einerseits an dem Bildschirm 4 z.B. über ein durch den Pfeil 24 dargestelltes analoges oder digitales Kabel übertragen und andrerseits dem Down-Scaler 13 entsprechend dem Pfeil 25 zugeführt. Die von dem Down-Scaler 13 reduzierte Datenstrom wird entsprechend dem Pfeil 26 dem digitalen Signalprozessor 14 zugeführt, der den Datenstrom mit entsprechenden Kompressionsverfahren enkodiert.

Der komprimierte Datenstrom wird entsprechend dem Pfeil 27 von der CPU 15 weiterverarbeitet, die zugleich alle Komponenten des Moduls 10 steuert. Die Videosignale werden entsprechend den Pfeilen 28, 29 und 30 von der CPU 15 an den flüchtigen Speicher 16, den nicht flüchtigen Speicher 17 bzw. das optische Laufwerk 18 übertragen. Mit der CPU 15 können die in den Speichern 16, 17, 18 gespeicherten Videosignale entsprechend dem Pfeil 33 und 34 den Schnittstellen 19 bzw. 22 zugeführt werden.

Mit dem USB-Kabel 20 wird das Modul 10 an dem PC - basierenden System 2 angeschlossen. Damit stellt das USB-Kabel 20 ein Kommando-Interface für Befehle des PC - basierenden Systems 2, beispielsweise ausgelöst durch die Tastatur 5 des Diagnosegeräts 1, an das Modul 10 dar. Zugleich kann über das USB-Kabel 20 das PC - basierende System 2 auf Daten in den Speichern 16, 17 bzw. auf den Datenträger im optischen Laufwerk 18 des Moduls 10 zugreifen. Auch ist eine externe Steuerung des Moduls über die Netzwerkverbindung 32, den HUB/ Switch 31 und die Kabelverbindung 21 möglich.

Das Modul 10 weist zudem einen nicht dargestellten Eingang zur Aufzeichnung akustischer Signale in den Speicher 16 oder 17 bzw. auf dem Datenträger des optischen Laufwerks 18 auf, beispielsweise für die Aufnahme der Tonsignale einer Ultraschalluntersuchung.

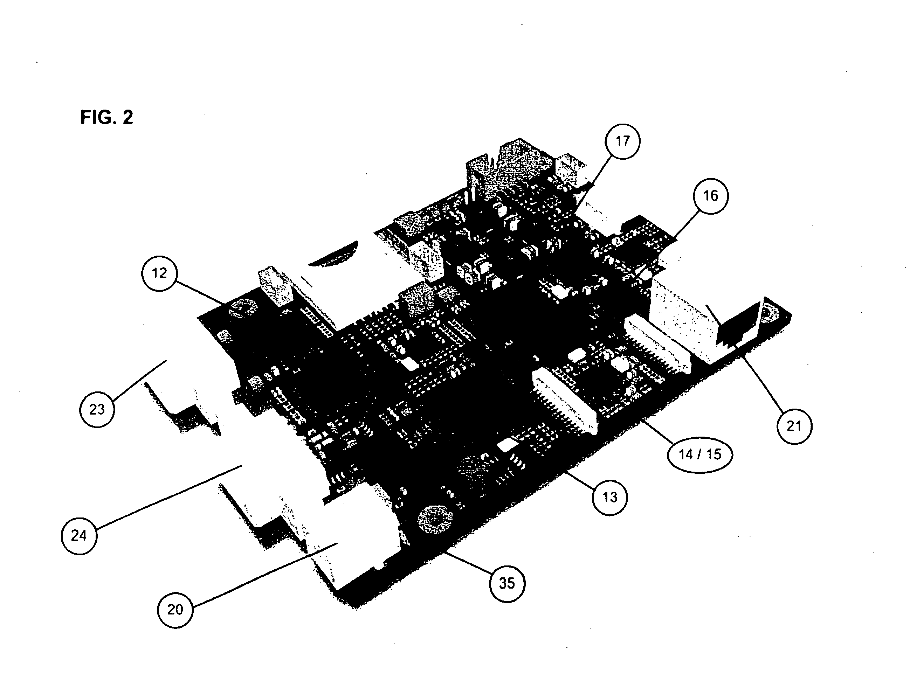

Gemäß Figur 2 ist in dem Gehäuse 3 des Moduls 10 (Figur 3) eine Leiterplatte 35 vorgesehen, die mit den einzelnen Komponenten des Moduls 10 bestückt ist, also dem Splitter 12, dem Down-Scaler 13, dem digitalen Signalprozessor 14 und CPU als weiteren Prozessor 15, welcher mit dem Signalprozessor 14 ein Teil bildet, den flüchtigen Speicher 16, den nicht flüchtigen Speicher 17 sowie den Anschlüssen für die Kabelverbindung 20, die Netzwerkverbindung 21, der Verbindung 23 der Graphikkarte 3 mit dem Splitter 12 und der Verbindung 24 des Splitters 2 mit dem Bildschirm 4.

Gemäß Figur 3 weist das Modul zudem einen USB-Stick-Anschluss 37 und einen Audio-Anschluss 38 auf. Das optische Laufwerk 18 ist dabei als Slimline-Laufwerk ausgebildet.

## Patentansprüche

1. Bildgebendes Diagnosegerät (1) mit einem PC (2), einer Grafikkarte (3), einem Schacht (9) zur Aufnahme eines optischen Laufwerks, einer Schnittstelle (6) zum Anschluss einer Untersuchungsvorrichtung (7) und einem Bildschirm (4) zur Darstellung der mit der Untersuchungsvorrichtung (7) erhaltenen Signale als bewegte Bilder, **dadurch gekennzeichnet, dass** in dem Schacht (9) des PC (2) zur Aufnahme eines optischen Laufwerks ein Modul (10) angeordnet ist, das einen Splitter (12), einen digitalen Signalprozessor (14) zur Datenreduktion und wenigstens einen Speicher (16, 17, 18) aufweist, wobei die Grafikkarte (3) mit dem Splitter (12) verbunden ist und die von der Grafikkarte (3) des PC (2) an den Splitter (12) übertragenen Daten von dem Splitter (12) einerseits dem Bildschirm (4) und andererseits dem digitalen Signalprozessor (14) zugeführt werden, von dem der reduzierte Datenstrom an den Speicher (16, 17, 18) zur Aufzeichnung der bewegten Bilder übertragen wird.

2. Diagnosegerät nach Anspruch 1, **dadurch gekennzeichnet, dass** das Modul (10) einen an den Splitter (12) angeschlossenen Down Sealer (13) zur Reduktion des an den digitalen Signalprozessor (14) übertragenen Datenstroms aufweist.

3. Diagnosegerät nach Anspruch 1 oder 2, **dadurch gekennzeichnet, dass** ein weiterer Prozessor (15) vorgesehen ist, der die von dem digitalen Signalprozessor (14) reduzierten Datenstrom zur Aufzeichnung durch den Speicher (16, 17, 18) weiter verarbeitet.

4. Diagnosegerät nach Anspruch 2 oder 3, **dadurch gekennzeichnet, dass** der weitere Prozessor (15) durch einen Teil des digitalen Signalprozessors (14) gebildet wird.

5. Diagnosegerät nach einem der vorstehenden Ansprüche, **dadurch gekennzeichnet, dass** der Speicher (16, 17, 18) ein flüchtiger und/oder nicht flüchtiger Speicher und/oder ein Datenträger ist.

6. Diagnosegerät nach Anspruch 5, **dadurch gekennzeichnet, dass** das Modul (10) ein Laufwerk (18) für den optischen Datenträger als Speicher aufweist.

7. Diagnosegerät nach Anspruch 6, **dadurch gekennzeichnet, dass** das Laufwerk (18) ein Slimline-Laufwerk ist.

8. Diagnosegerät nach einem der vorstehenden Ansprüche, **dadurch gekennzeichnet, dass** das Modul (10) eine Schnittstelle (19) zum Anschluss des Moduls (10) an den PC (2) und/oder an ein Ethernet aufweist.

9. Diagnosegerät nach Anspruch 5 und 8, **dadurch gekennzeichnet, dass** die Schnittstelle (19) zur Verbindung mit dem flüchtigen und/oder nicht flüchtigen Speicher (16, 17) oder mit dem optischen Laufwerk (18) ausgebildet ist.

10. Diagnosegerät nach Anspruch 8 oder 9, **dadurch gekennzeichnet, dass** die Schnittstelle (19) zur Verbindung durch ein serielles Bussystem (10) mit dem PC (2) ausgebildet ist.

11. Diagnosegerät nach einem der vorstehenden Ansprüche, **dadurch gekennzeichnet, dass** das Modul (10) einen Eingang zur Aufzeichnung akustischer Signale mit dem Speicher (16, 17, 18) aufweist.

12. Diagnosegerät nach einem der vorstehenden Ansprüche, **dadurch gekennzeichnet, dass** das Modul (10) so ausgebildet ist, dass es dem PC (2) über eine Schnittstelle (20) vorgibt, ein USB-Mass Storage Device oder ein natives DVD-Laufwerk zu sein, wodurch ermöglicht wird, den PC (2) über die in das Modul (10) eingelegte DVD zu booten.

13. Diagnosegerät nach einem der vorstehenden Ansprüche, **dadurch gekennzeichnet, dass** die mit dem Modul (10) gewonnenen Bilddaten online über ein Netzwerk übertragbar sind.

14. Modul (10) für ein bildgebendes Diagnosegerät (1) mit einem PC (2), welcher eine Grafikkarte (3), einen Schacht (9) zur Aufnahme eines optischen Laufwerks, eine Schnittstelle (6) zum Anschluss einer Untersuchungsvorrichtung (7) und einen Bildschirm (4) zur Darstellung der mit der Untersuchungsvorrichtung (7) erhaltenen Signale als bewegte Bilder aufweist, **dadurch gekennzeichnet, dass** das Modul (10) zur Aufnahme in den für das optische Laufwerk vorgesehenen Schacht (9) des PC (2) ausgebildet ist und einen Splitter (12), einen digitalen Signalprozessor (14) zur Datenreduktion und wenigstens einen Speicher (16, 17, 18) aufweist, wobei der Splitter (12) einerseits mit der Grafikkarte (3) des PC (2) und andererseits mit dem Bildschirm (4) des PC (2) verbindbar ist und der Splitter (12) an den Signalprozessor (14) und der Signalprozessor (14) an den Speicher (16, 17, 18) zur Aufzeichnung der bewegten Bilder angeschlossen ist.

## Geänderte Patentansprüche

### Geänderte Patentansprüche gemäss Regel 137(2) EPÜ.

**1.** Bildgebendes Diagnosegerät (1) mit einem PC (2), einer Grafikkarte (3), einem Schacht (9) zur Aufnahme eines optischen Laufwerks, einer Schnittstelle (6) zum Anschluss einer Untersuchungsvorrichtung (7) und einem Bildschirm (4) zur Darstellung der mit der Untersuchungsvorrichtung (7) erhaltenen Signale als bewegte Bilder, **dadurch gekennzeichnet, dass** in dem Schacht (9) des PC (2) zur Aufnahme eines optischen Laufwerks ein Modul (10) angeordnet ist, das einen Splitter (12), einen digitalen Signalprozessor (14) zur Datenreduktion und wenigstens einen Speicher (16, 17, 18) aufweist, wobei die Grafikkarte (3) mit dem Splitter (12) verbunden ist und die von der Grafikkarte (3) des PC (2) an den Splitter (12) übertragenen Daten von dem Splitter (12) einerseits dem Bildschirm (4) und andererseits dem digitalen Signalprozessor (14) zugeführt werden, von dem der reduzierte Datenstrom an den Speicher (16,17,18) zur Aufzeichnung der bewegten Bilder übertragen wird.

**2.** Diagnosegerät nach Anspruch 1, **dadurch gekennzeichnet, dass** das Modul (10) einen an den Splitter (12) angeschlossenen Down Sealer (13) zur Reduktion des an den digitalen Signalprozessor (14) übertragenen Datenstroms aufweist.

**3.** Diagnosegerät nach Anspruch 1 oder 2, **dadurch gekennzeichnet, dass** ein weiterer Prozessor (15) vorgesehen ist, der die von dem digitalen Signalprozessor (14) reduzierten Datenstrom zur Aufzeichnung durch den Speicher (16, 17, 18) weiter verarbeitet.

**4.** Diagnosegerät nach Anspruch 2 oder 3, **dadurch gekennzeichnet, dass** der weitere Prozessor (15) durch einen Teil des digitalen Signalprozessors (14) gebildet wird.

**5.** Diagnosegerät nach einem der vorstehenden Ansprüche, **dadurch gekennzeichnet, dass** der Speicher (16,17,18) ein flüchtiger und/oder nicht flüchtiger Speicher und/oder ein Datenträger ist.

**6.** Diagnosegerät nach Anspruch 5, **dadurch gekennzeichnet, dass** das Modul (10) ein Laufwerk (18) für den optischen Datenträger als Speicher aufweist.

**7.** Diagnosegerät nach Anspruch 6, **dadurch gekennzeichnet, dass** das Laufwerk (18) ein Slimline-Laufwerk ist.

**8.** Diagnosegerät nach einem der vorstehenden Ansprüche, **dadurch gekennzeichnet, dass** das Modul (10) eine Schnittstelle (19) zum Anschluss des Moduls (10) an den PC (2) und/oder an ein Ethernet aufweist.

**9.** Diagnosegerät nach Anspruch 5 und 8, **dadurch gekennzeichnet, dass** die Schnittstelle (19) zur Verbindung mit dem flüchtigen und/oder nicht flüchtigen Speicher (16,17) oder mit dem optischen Laufwerk (18) ausgebildet ist.

**10.** Diagnosegerät nach Anspruch 8 oder 9, **dadurch gekennzeichnet, dass** die Schnittstelle (19) zur Verbindung durch ein serielles Bussystem (10) mit dem PC (2) ausgebildet ist.

**11.** Diagnosegerät nach einem der vorstehenden Ansprüche, **dadurch gekennzeichnet, dass** das Modul (10) einen Eingang zur Aufzeichnung akustischer Signale mit dem Speicher (16,17,18) aufweist.

**12.** Diagnosegerät nach einem der vorstehenden Ansprüche, **dadurch gekennzeichnet, dass** das Modul (10) so ausgebildet ist, dass es dem PC (2) über eine Schnittstelle (20) vorgibt, ein USB-Mass Storage Device oder ein natives DVD-Laufwerk zu sein, wodurch ermöglicht wird, den PC (2) über die in das Modul (10) eingelegte DVD zu booten.

**13.** Diagnosegerät nach einem der vorstehenden Ansprüche, **dadurch gekennzeichnet, dass** die mit dem Modul (10) gewonnenen Bilddaten online über ein Netzwerk übertragbar sind.
